# EUROPEAN PATENT APPLICATION

(11) **EP 4 640 139 A2**
(43) Date of publication of application: **29.10.2025**
(21) Application number: 24826072.1
(22) Date of filing: 22.03.2024
(51) Int. Cl.: A61B 5/00, H04N 5/58, G06V 40/16, G16H 10/60

(54) **DISPLAY DEVICE AND METHOD FOR CONTROLLING SAME**

(30) Priority: 20.06.2023 KR 20230079318
(71) Applicant: Samsung Electronics Co., Ltd., Suwon-si, Gyeonggi-do 16677 (KR)
(72) Inventor: PARK, Jaesung, Suwon-si Gyeonggi-do 16677 (KR); MOON, Youngsu, Suwon-si Gyeonggi-do 16677 (KR); JEONG, Younghoon, Suwon-si Gyeonggi-do 16677 (KR)
(74) Representative: Appleyard Lees IP LLP
(86) International application number: PCT/KR2024/003586
(87) International publication number: WO 2024/262748

(57) **Abstract**

A display device includes: a display; communication circuitry configured to communicate with an external device; and at least one memory storing one or more instructions; and at least one processor configured to execute the one or more instructions, wherein the one or more instructions, when executed by the at least one processor, cause the display device to: display an image on the display based on image information received through the communication circuitry, identify, in the image, one or more feature points of a patient and an omega shape of the patient, identify, based on the one or more feature points and the omega shape, whether the image may include a predetermined detection area associated with biometric information of the patient, and based on identifying that the image may include the predetermined detection area, adjust a display parameter of at least one of the display or the image.

## Description

### [Technical Field]

The disclosure relates to a display device that may correct an image and identify a predetermined area from the corrected image.

### [Background Art]

**In** general, feature points in a human image are used in various technical fields. **In** particular, feature points detected from an image of a human face are useful in face recognition technology to determine an identity of an object in the image. Such feature points may also be used in the medical field.

With the development of science and technology, technologies such as telehealth or telemedicine, or home-care capable of diagnosing a patient's health state or abnormalities by analyzing only the patient's image from a distance without a face-to-face meeting between the patient and doctor, are being developed.

In order to make a diagnosis from a patient's image, accurate detection of feature points in the image is critical.

However, when a patient's image is captured, a dark image of the patient may be obtained depending on lighting conditions of an indoor environment where the patient is located, or a backlit image of the patient may be obtained depending on a position of a camera and lighting relative to the patient. As a result, detection of feature points in the patient's image may be inaccurate, and identification of an area for detecting vital signs in the patient's image may not be smooth.

In addition, even when a patient's face has a dark skin tone or is tilted, an area for detecting vital signs in the patient's image may not be smoothly identified, resulting in inaccuracies in diagnosing and treating patients via images during telemedicine.

### [Disclosure]

Provided are a display device that may identify a predetermined detection area by correcting a brightness of a screen or an image, and a method for controlling the same.

According to an aspect of the disclosure, a display device includes: a display; communication circuitry configured to communicate with an external device; and at least one memory storing one or more instructions; and at least one processor configured to execute the one or more instructions, wherein the one or more instructions, when executed by the at least one processor, cause the display device to: display an image on the display based on image information received through the communication circuitry, identify, in the image, one or more feature points of a patient and an omega shape of the patient, identify, based on the one or more feature points and the omega shape, whether the image may include a predetermined detection area associated with biometric information of the patient, and based on identifying that the image may include the predetermined detection area, adjust a display parameter of at least one of the display or the image.

The one or more instructions, when executed by the at least one processor, may further cause the display device to: identify a center pole of the image, identify, in the image, a left side of the patient and a right side of the patient based on the center pole, the omega shape and the one or more feature points, identify whether a left feature point on the left side of the patient is included in the one or more feature points, identify whether a right feature point on the right side of the patient is included in the one or more feature points, and based on identifying that only one of the left feature point and the right feature point is included in the one or more feature points, map a feature point from among the left feature point and the right feature point that is included in the one or more feature points to a corresponding opposite side of the image.

The one or more instructions, when executed by the at least one processor, may further cause the display device to: based on identifying that at least one of the omega shape or the one or more feature points of the patient is not included in the image, control the communication circuitry to transmit, to the external device, environment guide information, and the environment guide information may include information related to an adjustment of a location of the patient and a posture of the patient.

The one or more instructions, when executed by the at least one processor, may further cause the display device to adjust the display parameter of the image based on illuminance information received through the communication circuitry and predetermined reference illuminance information.

The one or more instructions, when executed by the at least one processor, may further cause the display device to control the communication circuitry to transmit environment guide information about illuminance adjustment to the external device, based on illuminance information received through the communication circuitry and predetermined reference illuminance information.

The one or more instructions, when executed by the at least one processor, may further cause the display device to, based on identifying that a brightness of the image is non-uniform, perform pre-processing on the image to make the brightness of the image uniform.

The at least one memory further stores reference biometric information of the patient, and the one or more instructions, when executed by the at least one processor, may further cause the display device to, based on receiving medical care request information through the communication circuitry: detect the biometric information of the patient from the predetermined detection area, obtain diagnosis result information based on the detected biometric information and the stored reference biometric information, and control the communication circuitry to transmit the obtained diagnosis result information to the external device.

The one or more instructions, when executed by the at least one processor, may further cause the display device to: based on identifying the omega shape and not identifying the one or more feature points, identify whether the image information was obtained in a backlit environment based on brightness information of the image information, and based on identifying that the image information was obtained in the backlit environment, adjust the display parameter of the image and perform pre-processing on the image to make a brightness of the image uniform.

The one or more instructions, when executed by the at least one processor, may further cause the display device to, based on identifying the omega shape and not identifying the one or more feature points: obtain illuminance information from the external device through the communication circuitry, identify whether an illuminance of a space where the external device is located is less than a first reference illuminance and greater than or equal to a second reference illuminance based on the illuminance information, first reference illuminance information and second reference illuminance information, based on the illuminance of the space being less than the first reference illuminance and greater than or equal to the second reference illuminance, identify that the image information was obtained in the backlit environment, and based on the illuminance of the space being less than the second reference illuminance, transmit environment guide information to the external device, wherein the second reference illuminance is less than the first reference illuminance, and the environment guide information may include information related to an adjustment to the illuminance of the space.

The one or more feature points may include one or more feature points of a face of the patient, the one or more feature points of the face include a pair of left eye endpoints, a pair of right eye endpoints, a pair of mouth endpoints, and a nose tip point, and the predetermined detection area may include an orbital area of the face and an infraorbital area of the face.

The one or more instructions, when executed by the at least one processor, may further cause the display device to: obtain a skin reflection coefficient of the patient based on first image information obtained in an illuminated environment and second image information obtained in an unilluminated environment, the first image information and the second image information being received from the external device, and transmit illuminance information corresponding to the skin reflection coefficient to the external device.

According to an aspect of the disclosure, a method for controlling a display device, includes: identifying an omega shape of a patient and one or more feature points of the patient based on image information received from an external device through a communication circuitry; based on identifying the omega shape and the one or more feature points, identifying whether the image information may include a detection area associated with biometric information of the patient; and based on identifying the detection area, adjusting a display parameter of at least one of the image information or a display of the display device.

The method may further include: based on not identifying the one or more feature points and the omega shape in the image information, transmitting first environment guide information to the external device, wherein the first environment guide information may include information related to an adjustment of a location of the patient and a posture of the patient; and based on identifying the omega shape in the image information and not identifying the one or more feature points in the image information: identifying an environment of a space where the external device is located as a backlit environment, adjusting the display parameter of the display, and performing pre-processing on the image information to make a brightness of an image displayed on the display uniform, and transmitting second environment guide information to the external device based on reference illuminance information and illuminance information received from the external device through the communication circuitry, wherein the second environment guide information may include information related to an adjustment to an illuminance of the space.

The method may further include: identify a center pole of the image information; identifying, in the image information, a left side of the patient and a right side of the patient based on the center pole; identifying whether the one or more feature points may include a left feature point on the left side of the patient, and identifying whether the one or more feature points may include a right feature point on the right side of the patient; based on identifying that only one of the left feature point and the right feature point is included in the one or more feature points: identifying a feature point, among the left feature point and the right feature point, included in the one or more feature points as a detected feature point, and identifying a feature point, among the left feature point and the right feature point, not included in the one or more feature points as a missing feature point; generating the missing feature point by mapping the detected feature point to a side from among the left side of the patient and the right side of the patient corresponding to the missing feature point; and identifying the detection area based on the generated feature point and the detected feature point.

The method may further include: obtaining a skin reflection coefficient of the patient based on first image information obtained in an illuminated environment and second image information obtained in an unilluminated environment, the first image information and the second image information being received from the external device, and transmitting illuminance information corresponding to the skin reflection coefficient to the external device.

According to one or more embodiments of the disclosure, feature points of a body may be easily and accurately detected by correcting an image obtained in a low-light environment or backlight environment.

According to one or more embodiments of the disclosure, in a state where an illuminance of an obtained image is non-uniform, the obtained image may be corrected to easily and accurately detect feature points.

According to one or more embodiments of the disclosure, when a reflectance is low due to a dark skin tone of a person, by obtaining an image of the person after adjusting a brightness of a screen or a brightness of a lighting of a display device, feature points of the person's body may be easily and accurately detected from the obtained image, and a predetermined detection area which is a vital sign detection area may be easily identified.

According to one or more embodiments of the disclosure, when a feature point of a body part is not detected from an image of the body, or when a person's head posture changes, a vital sign detection area may be easily identified by detecting an omega shape.

According to one or more embodiments of the disclosure, a performance and accuracy of diagnosis may be improved by identifying a detection area of a vital sign.

### [Description of Drawings]

The above and other aspects, features, and advantages of certain embodiments of the present disclosure will be more apparent from the following description taken in conjunction with the accompanying drawings, in which:
FIG. 1 illustrates a configuration of a display system including a first display device and a second display device according to an embodiment;
FIG. 2 is a control block diagram of a first display device according to an embodiment;
FIG. 3 illustrates an example of detection of feature points by a first display device according to an embodiment;
FIG. 4 illustrates an example of detection of an omega shape by a first display device according to an embodiment;
FIGS. 5A, 5B and 5C illustrate examples of generation of feature points and identification of predetermined detection areas by a first display device according to an embodiment;
FIG. 6 is a control block diagram of a second display device according to an embodiment;
FIGS. 7A and 7B illustrate examples of obtaining a skin reflection coefficient by a second display device according to an embodiment;
FIG. 8 is a flowchart of a method for controlling a first display device according to an embodiment;
FIG. 9 is a control block diagram of a display device according to another embodiment; and
FIGS. 10A and 10B illustrate examples of detection of body feature points by a display device according to another embodiment.

### [Modes of the Disclosure]

Various embodiments of the disclosure and terms used therein are not intended to limit the technical features described in the disclosure to particular embodiments, and it should be construed as including various modifications, equivalents, or alternatives of a corresponding embodiment.

With regard to description of drawings, similar reference numerals may be used for similar or related components.

A singular form of a noun corresponding to an item may include one item or a plurality of the items unless context clearly indicates otherwise.

As used herein, each of the expressions "A or B," "at least one of A and B," "at least one of A or B," "A, B, or C," "at least one of A, B, and C," and "at least one of A, B, or C," may include one or all possible combinations of the items listed together with a corresponding expression among the expressions.

It will be understood that the terms "first", "second", etc., may be used only to distinguish one component from another, not intended to limit the corresponding component in other aspects (e.g., importance or order).

It is said that one (e.g., first) component is "coupled" or "connected" to another (e.g., second) component, with or without the terms "functionally" or "communicatively". When referenced, it means that one component may be connected to the other component directly (e.g., by wire), wirelessly, or through a third component.

It will be understood that when the terms "includes," "comprises," "including," and/or "comprising," when used in this specification, specify the presence of stated features, figures, steps, operations, components, members, or combinations thereof, but do not preclude the presence or addition of one or more other features, figures, steps, operations, components, members, or combinations thereof.

An expression that one component is "connected", "coupled", "supported", or "in contact" with another component includes a case in which the components are directly "connected", "coupled", "supported", or "in contact" with each other and a case in which the components are indirectly "connected", "coupled", "supported", or "in contact" with each other through a third component.

It will also be understood that when one component is referred to as being "on" or "over" another component, it may be directly on the other component or intervening components may also be present.

The term "and/or" includes any and all combinations of one or more of a plurality of associated listed items.

Hereinafter, an operation principle and embodiments of the disclosure will be described in detail with reference to the accompanying drawings.

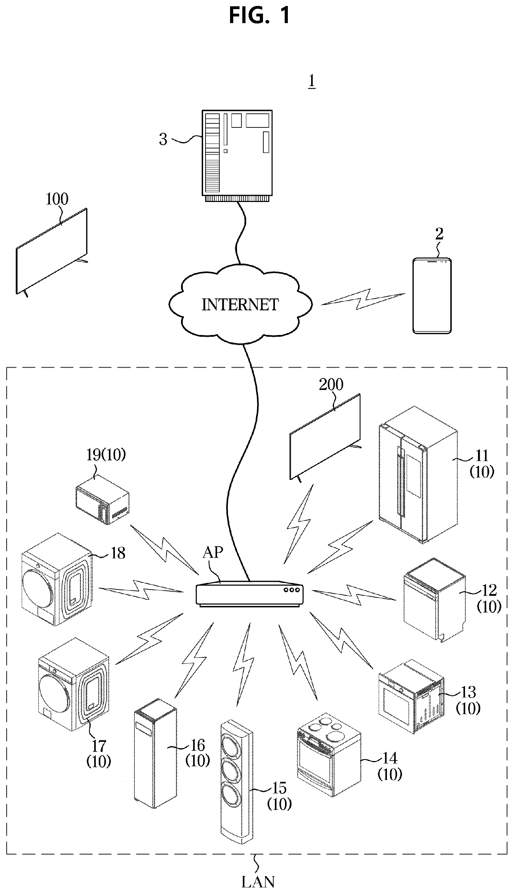

FIG. 1 illustrates a configuration of a display system 1 including a first display device and a second display device according to an embodiment.

The display system 1 may be implemented in a cloud manner over an Internet network established online.

The display system 1 may include two or more display devices 100 and 200 located in different spaces.

The display system 1 may further include a user device 2 and a server 3.

Hereinafter, the display system including two display devices will be described. To distinguish the two display devices, the two display devices will be referred to as a first display device 100 and a second display device 200.

The first display device 100 may be a device in a medical institution, and the second display device 200 may be a device in a home or office.

The first and second display devices 100 and 200 may each include an image sensor and a display which displays a screen. The first and second display devices 100 and 200 may further each include a microphone and a speaker. The first and second display devices 100 and 200 may further each include a lighting.

The first display device 100 may be a television or a user device.

The first display device 100 may include a communication module for communicating with the user device 2 or the server 3, a user interface for receiving user input or outputting information to a user, at least one processor for controlling an operation of the first display device 100, and at least one memory storing a program for controlling an operation of the first display device 100.

The second display device 200 may be a home appliance or a user device.

The second display device 200 may include a communication module for communicating with another home appliance 10, the user device 2, or the server 3, a user interface for receiving user input or outputting information to a user, at least one processor for controlling an operation of the second display device 200, and at least one memory for storing a program for controlling an operation of the second display device 200.

The other home appliance 10 may be at least one of various types of home appliances. For example, as shown in the accompanying drawings, the home appliance 10 may include a refrigerator 11, a dishwasher 12, an electric range 13, an electric oven 14, an air conditioner 15, a clothing care apparatus 16, a washing machine 17, a dryer 18, and a microwave oven 19, without being limited thereto. For example, the other home appliance 10 may include various types of appliances such as a cleaning robot, a vacuum cleaner, a television, and the like.

Furthermore, the aforementioned home appliances are only examples, and in addition to the aforementioned home appliances, other appliances connected to other home appliances, the user device 2, or the server 3 to perform operations described below may be included in the second display device 200 according to an embodiment.

The server 3 may include a communication module communicating with another server, the first and second display devices 100 and 200, or the user device 2, at least one processor processing data received from the other server, the first and second display devices 100 and 200 or the user device 2, and at least one memory storing processed data and/or programs for processing data.

The server 3 may be implemented as a variety of computing devices, such as a workstation, a cloud, a data drive, a data station, and the like. The server 3 may be implemented as one or more servers physically or logically separated based on a function, detailed configuration of function, or data, and may transmit and receive data through communication between servers and process the transmitted and received data.

The server 3 may perform functions such as managing a user account, registering the first and second display devices 100 and 200 in association with the user account, managing or controlling the registered first and second display devices 100 and 200, and the like. For example, a user may access the server 3 via the user device 2 and may generate a user account. The user account may be identified by an identifier (ID) and a password set by the user. Here, the user may be a doctor associated with the first display device 100 and may be a patient associated with the second display device 200.

The server 3 may register the first and second display devices 100 and 200 to the user account according to a predetermined procedure. For example, the server 3 may link identification information of the first and second display devices 100 and 200 (such as a serial number or MAC address) to the user account to register, manage, and control the first and second display devices 100 and 200.

The user device 2 may include a communication module capable of communicating with the first display device 100, the second display device 200 or the server 3, a user interface receiving a user input or outputting information to a user, at least one processor controlling an operation of the user device 2, and at least one memory storing a program for controlling the operation of the user device 2.

The user device 2 may be carried by a user, or placed in a user's home or office, a medical institution, or the like. The user device 2 may include a personal computer, a terminal, a portable telephone, a smartphone, a handheld device, a wearable device, and the like, without being limited thereto.

The memory of the user device 2 may store a program for controlling the first display device 100 or the second display device 200, i.e. an application. The application may be pre-installed on the user device 2, or may be downloaded from an external server for installation.

By executing the application installed on the user device 2 by a user, the user may access the server 3, generate a user account, and perform communication with the server 3 based on the login user account to register the first display device 100 or the second display device 200.

For example, by operating the first display device 100 to enable the first display device 100 to access the server 3 according to a procedure guided by the application installed on the user device 2, the server 3 may register the first display device 100 with the user account by assigning the identification information (such as a serial number or MAC address) of the first display device 100 to the corresponding user account.

By operating the second display device 200 to enable the second display device 200 to access the server 3 according to a procedure guided by the application installed on the user device 2, the server 3 may register the second display device 200 with the user account by assigning the identification information (such as a serial number or MAC address) of the second display device 200 to the corresponding user account.

A user may control the first display device 100 using the application installed on the user device 2. For example, by logging into a user account with the application installed on the user device 2, the first display device 100 registered in the user account appears, and by inputting a control command for the first display device 100, the control command may be delivered to the first display device 100 via the server 3.

A user may control the second display device 200 using the application installed on the user device 2. For example, by logging into a user account with the application installed on the user device 2, the second display device 200 registered in the user account appears, and by inputting a control command for the second display device 200, the control command may be delivered to the second display device 200 via the server 3.

A network may include both a wired network and a wireless network. The wired network may include a cable network or a telephone network, and the wireless network may include any networks transmitting and receiving a signal via radio waves. The wired network and the wireless network may be connected to each other.

The network may include a Wide Area Network (WAN) such as the Internet, a Local Area Network (LAN) formed around an Access Point (AP), and a short range wireless network not using an AP. The short range wireless network may include Bluetooth^{™} (IEEE 802.15.1), Zigbee (IEEE 802.15.4), Wi-Fi Direct, Near Field Communication (NFC), and Z-Wave, without being limited thereto.

The AP may connect the first and second display devices 100 and 200 or the user device 2 to a WAN connected to the server 3. The first and second display devices 100 and 200 or the user device 2 may be connected to the server 3 via a WAN.

The AP may communicate with the second display device 200 or the user device 2 using wireless communication such as Wi-Fi^{™} (IEEE 802.11), Bluetooth^{™} (IEEE 802.15.1), Zigbee (IEEE 802.15.4), etc., and access a WAN using wired communication, without being limited thereto.

According to various embodiments, the first and second display devices 100 and 200 may be directly connected to the user device 2 or the server 3 without going through an AP.

The first and second display devices 100 and 200 may be connected to the user device 2 or the server 3 via a long range wireless network or a short range wireless network.

For example, the first and second display devices 100 and 200 may be connected to the user device 2 via a short range wireless network (e.g., Wi-Fi Direct).

In another example, the first and second display devices 100 and 200 may be connected to the user device 2 or the server 3 via a WAN using a long range wireless network (e.g., a cellular communication module).

In still another example, the first and second display devices 100 and 200 may access a WAN using wired communication, and may be connected to the user device 2 or the server 3 via a WAN.

Upon accessing a WAN using wired communication, the second display device 200 may also act as an access point. Accordingly, the second display device 200 may connect other home appliances 10 to a WAN to which the server 3 is connected.

The second display device 200 may transmit information about an operation or state to the user device 2 or the server 3 via the network. For example, the second display device 200 may transmit information about an operation or state to the user device 2 or the server 3 upon receiving a request from the server 3, in response to an event in the second display device 200, or periodically or in real time.

**In** response to receiving the information about the operation or state from the first display device 100 or the second display device 200, the server 3 may update the stored information about the operation or state of the first display device 100 or the second display device 200, and transmit the updated information about the operation and state of the first display device 100 or the second display device 200 to the user device 2 via the network. Here, updating the information may include various operations in which existing information is changed, such as adding new information to the existing information, replacing the existing information with new information, and the like.

The first display device 100 or the second display device 200 may obtain various information from the user device 2 or the server 3, and may provide the obtained information to a user. For example, the first display device 100 or the second display device 200 may obtain information related to a function of the first display device 100 or the second display device 200 (e.g., non-face-to-face medical care, etc.) and various environment information (e.g., illuminance, etc.) from the server 3, and may output the obtained information via a user interface.

The first display device 100 or the second display device 200 may operate according to a control command received from the user device 2 or the server 3. For example, the first display device 100 or the second display device 200 may operate in accordance with a control command received from the server 3, based on a prior authorization obtained from a user to operate in accordance with the control command of the server 3 even without a user input. Here, the control command received from the server 3 may include a control command input by the user via the user device 2 or a control command based on preset conditions, without being limited thereto.

The user device 2 may transmit information about a user to the first display device 100 or the second display device 200 or the server 3 through the communication module. For example, the user device 2 may transmit information about a user's location, a user's health state, a user's preference, a user's schedule, etc. to the server 3. The user device 2 may transmit information about the user to the server 3 based on the user's prior authorization.

The first display device 100 or the second display device 200, the user device 2, or the server 3 may use techniques such as artificial intelligence to determine a control command. For example, the server 3 may receive information about an operation or a state of the first display device 100 or the second display device 200 or information about a user of the user device 2, process the received information using techniques such as artificial intelligence, and transmit a processing result or a control command to the first display device 100 or the second display device 200 or the user device 2 based on the processing result.

Hereinafter, the first and second display devices according to various embodiments will be described in detail with reference to the drawings.

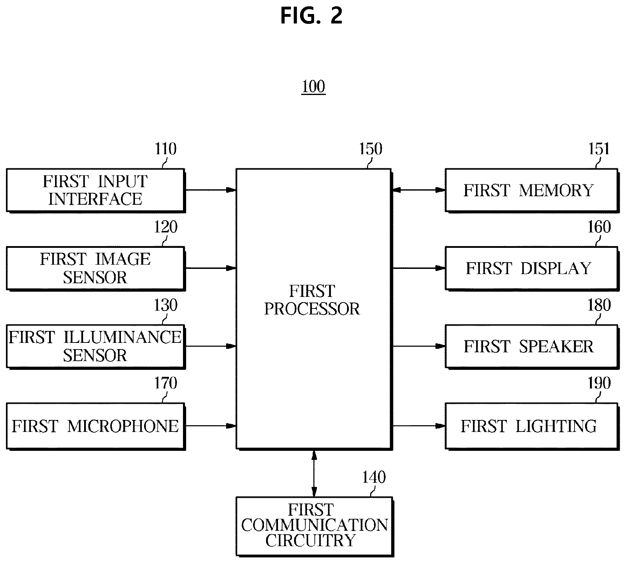

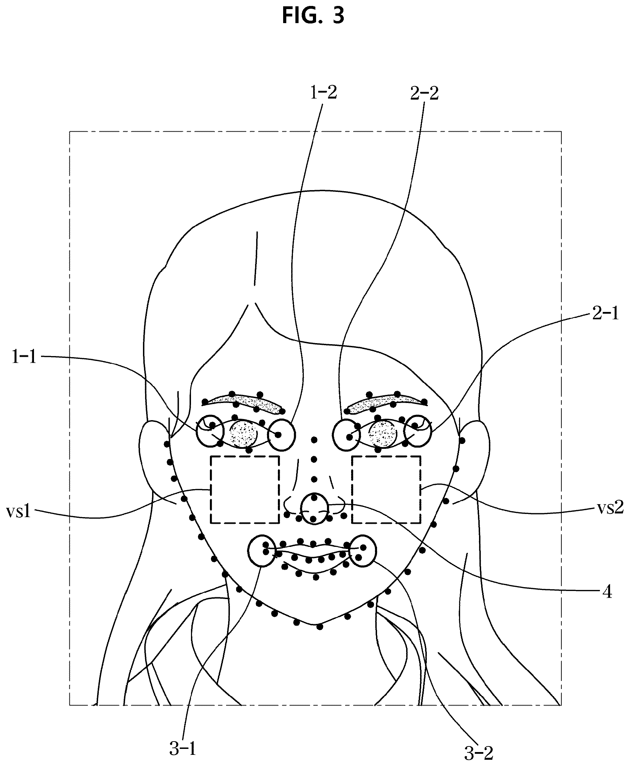

FIG. 2 is a control block diagram of a first display device according to an embodiment, which is described with reference to FIG. 3, FIG. 4, FIG. 5A, FIG. 5B and FIG. 5C.

The first display device 100 may include a first input interface 110, a first image sensor 120, a first illuminance sensor 130, a first communication circuitry 140, a first processor 150, a first memory 151, and a first display 160, and may further include a first microphone 170, a first speaker 180, and a first lighting 190.

The first input interface 110 receives a user input and transmits the received user input to the first processor 150. Here, the user may be a medical professional such as a doctor or nurse.

The user input may further include a power-on command and a power-off command.

The user input may include an on command and an off command for non-face-to-face medical care.

The user input may include a first command to collect biometric information of a patient in a healthy state, and a second command to collect biometric information of a patient seeking medical care.

The biometric information of a patient in a normal state may be reference biometric information for diagnosing a patient.

The first input interface 110 may receive patient identification information, a patient registration command, medical care information, and diagnosis result information.

The first input interface 110 may receive an on command, an off command, and a sensing command of the first image sensor 120.

The sensing command of the first image sensor 120 may be either the first command or the second command.

The first input interface 110 may receive an on command and an off command of the first lighting 190.

The first input interface 110 may be physically separate from the first display 160.

The first input interface 110 may include a hardware device such as various buttons, switches, a pedal, a keyboard, a mouse, a track-ball, various levers, handles, and sticks.

The first input interface 110 may also include a Graphical User Interface (GUI) such as a touch pad, i.e., a software device. The touch pad is implemented as a Touch Screen Panel (TSP) and may form a mutual layer structure with the display.

The first image sensor 120 may obtain an image of a person around the first display device 100, convert image information about the obtained image into an electrical image signal, and transmit the converted image signal to the first processor 150. The person around the first display device 100 may be a doctor or a patient.

The first image sensor 120 may include a Charge-Coupled Device (CCD) image sensor or Complementary Metal Oxide Semiconductor (CMOS) image sensor.

The first image sensor 120 may include a camera. The first image sensor 120 may include a three-dimensional (3D) space recognition sensor such as a time-of-flight (TOF) camera, stereo camera, etc.

The first illuminance sensor 130 may detect an illuminance around the first image sensor 120, and transmit illuminance information about the detected illuminance to the first processor 150.

The first image sensor 120 and the first illuminance sensor 130 may be physically separate from the first display 160.

The first communication circuitry 140 may include at least one constituent component enabling communication between components of the first display device 100.

The first communication circuitry 140 may include at least one constituent component enabling communication with an external device, for example, at least one of a short-range communication module, a wired communication module, or a wireless communication module. Here, the external device may include the second display device 200.

The first communication circuitry 140 may include both a wired network and a wireless network.

The first processor 150 oversees control related to operations of the first display device 100. A single or a plurality of first processors 150 may be provided. That is, at least one first processor 150 may be provided.

The first processor 150 performs control related to operations of the first display device 100 using data stored in the first memory 151.

In response to receiving a patient registration command from the first input interface 110, the first processor 150 may control the first display 160 to display a patient information recording image for recording patient information.

Based on a user input received from the first input interface 110 while the patient information recording image is displayed on the first display 160, the first processor 150 may obtain patient information, and may control the first memory 151 to store the obtained patient information.

The patient information is patient identification information for identifying a patient, and may include a date of birth, name, address, contact information, and code information of the patient.

In response to receiving the first command from the first input interface 110, the first processor 150 may control the first image sensor 120 to be activated, may obtain reference biometric information of the patient based on image information of the patient obtained by the activated first image sensor 120, and may control the first memory 151 to store the obtained patient's reference biometric information.

The patient's image obtained in response to the first command may be a body image of the patient in a healthy state. The body may include at least one of a face, arms, legs, or torso.

The reference biometric information may include reference color information of the body and reference vital sign information of a predetermined detection area. For example, a vital sign may include tremor due to pulse or tremor due to spasm.

In obtaining the patient's reference biometric information, the first processor 150 may control the first lighting 190 based on preset environment information.

The preset environment may include an environment in which the first lighting 190 is turned off, an environment in which the first lighting 190 is turned on, or an environment with a preset illuminance in a first space where the first display device is located. The first space where the first display device 100 is located may be a space in which patients are treated in a medical institution.

The first processor 150 may control a brightness of the first lighting 190 to allow the first space to become an environment with the preset illuminance.

In obtaining the patient's reference biometric information, the first processor 150 may also control a brightness of the first display 160 to allow an illuminance of an indoor space to become the preset illuminance.

In response to receiving the second command from the first input interface 110, the first processor 150 may control the first lighting 190 based on the preset environment information, may control the first image sensor 120 to be activated, and then may obtain biometric information for diagnosis based on the patient's image information obtained by the activated first image sensor 120. In this case, the first processor 150 may obtain diagnosis information by comparing the reference biometric information and the biometric information for diagnosis, and may control the first display 160 to display the obtained diagnosis information.

In response to receiving medical care request information and patient identification information from the second display device 200, the first processor 150 may control the first display 160 to display the received medical care request information and patient identification information.

In response to receiving a medical care approval command through the first input interface 110, the first processor 150 may transmit the received medical care approval command and guide information for non-face-to-face medical care to the second display device 200.

In response to receiving the patient's image information from the second display device 200, the first processor 150 may control the first display 160 to display the received patient's image information.

The first processor 150 may control the first display 160 to display the patient identification information and diagnosis history information of the patient along with the received patient's image information.

The first processor 150 may obtain the biometric information for diagnosis based on the received patient's image information, and may control the first display 160 to display the obtained biometric information for diagnosis.

The first processor 150 may obtain reference biometric information corresponding to the patient identification information received from the second display device 200 among the information stored in the first memory 151, and may obtain diagnosis result information by comparing the received biometric information for diagnosis and the reference biometric information. Also, the first processor 150 may control the first display 160 to display the obtained diagnosis result information, and may transmit the diagnosis result information to the second display device 200.

The first processor 150 may obtain the diagnosis result information based on diagnostic criteria information stored in the first memory 151.

**In** displaying user's image information on the first display 160, the first processor 150 may adjust a display parameter of the image displayed on the first display 160, and may adjust a display parameter of the first display 160.

For example, the display parameter may include a brightness, saturation, contrast, and color of the first display 160, and may include a brightness, saturation, contrast, and color of the image.

The display parameter of the first display 160 may be an output parameter of the first screen.

**In** displaying the patient's image information on the first display 160, the first processor 150 may identify image information of a color patch from the patient's image information received from the second display device 200, and may obtain color information of the color patch based on the identified image information of the color patch. Also, the first processor 150 may obtain color correction information based on the obtained color information of the color patch and pre-stored color information of color patch, and may correct the patient's image information based on the obtained color correction information.

The first processor 150 may transmit the corrected image information of the patient to the second display device 200, thereby allowing the patient and doctor to view the same patient's image.

The first processor 150 may determine whether an illuminance is uniform based on the received patient's image information. Based on a determination that the illuminance in the image is non-uniform, the first processor 150 may correct the received patient's image information to allow the illuminance of the image to become uniform.

The first processor 150 may confirm illuminance information received from the second display device 200, and may correct a brightness of the received patient's image information based on the confirmed illuminance information. The illuminance information received from the second display device 200 is illuminance information of a second space where the patient is located together with the second display device 200, and may be detected by a second illuminance sensor 230.

The first processor 150 may also transmit, to the second display device 200, environment guide information for guiding rephotography to the second display device 200 based on the illuminance information received from the second display device 200 and the patient's image information.

The first processor 150 may perform diagnosis based on the patient's image information. Here, the patient's image information for diagnosis may be original image information transmitted from the second display device 200 or image information whose color has been corrected due to a color patch.

More specifically, in performing the diagnosis, the first processor 150 may detect feature points of the patient's body, and determine whether the body is left and right symmetric. Hereinafter, a face of the patient from among the patient's body is described as an example.

The first processor 150 may detect feature points for each face landmark based on a face image, and may perform labeling on the plurality of detected feature points. The face landmark may include eyes, nose, mouth, eyebrows, and chin.

That is, the plurality of feature points may include position information and labeling information.

As shown in FIG. 3, the first processor 150 may label a right endpoint of a right eye 1-1, a left endpoint of the right eye 1-2, a left endpoint of a left eye 2-1, a right endpoint of the left eye 2-2, a right endpoint of a mouth 3-1, a left endpoint of the mouth 3-2, and a nose tip point 4.

The first processor 150 may determine whether all feature points for each face landmark on the left and right sides of the face image have been detected based on the position information and labeling information for each feature point.

For example, the first processor 150 may determine whether the right endpoint of the right eye 1-1, the left endpoint of the right eye 1-2, and the right endpoint of the mouth 3-1 of the right face image have all been detected, and may determine whether the left endpoint of the left eye 2-1, the right endpoint of the left eye 2-2, and the left endpoint of the mouth 3-2 of the face image have all been detected.

The first processor 150 may determine whether all the left feature points of the left face image have been detected and whether all the right feature points of the right face image have been detected, based on labeling information of pre-stored left feature points of the left face image, labeling information of pre-stored right feature points of the right face image, and labeling information of the plurality of detected feature points.

As shown in FIG. 3, based on a determination that all the feature points of the left and right face images have been detected in the patient's image, the first processor 150 may identify detection areas of vital signs (vs1 and vs2, also referred to as "vital sign detection area") based on the feature points of the left and right face images in the patient's image.

The vital sign detection areas vs1 and vs2 may be predetermined detection areas.

The first processor 150 may determine whether an illuminance of the second space is greater than or equal to a first reference illuminance based on the illuminance information received from the second display device 200 and first reference illuminance information. Based on a determination that the illuminance of the second space is greater than or equal to the first reference illuminance and all the feature points of the left and right face images have been detected from the image received from the second display device 200, the first processor 150 may identify the vital sign detection areas based on the feature points of the left and right face images in the image.

The second space may be a space in which the second display device 200 is placed and the patient is located.

Based on a determination that at least one feature point among the feature points of the left and right face images has not been detected, the first processor 150 may determine whether an omega shape (Ω-Shape) has been detected based on the face image.

The first processor 150 may also determine whether the omega shape (Ω-Shape) has been detected based on the face image, along with detection of the feature points of the face image.

The first processor 150 may determine whether the face image is left-right symmetric based on the detected omega shape.

Based on a determination that the face image is not left-right symmetric, the first processor 150 may transmit, to the second display device 200, environment guide information that guides adjustment of a posture and location of the patient.

Determining that the face image is not left-right symmetric may include determining that the patient is not looking at the second image sensor 220.

Determining that the patient is not looking at the second image sensor 220 may include determining whether the second image sensor and the patient's face are facing each other.

For example, the first processor 150 may obtain a difference value between a size of the left face image and a size of the right face image based on a center pole, and based on the obtained size difference value being greater than or equal to a predetermined value, the first processor 150 may determine that the patient is not looking at the second image sensor 220.

The first processor 150 may determine whether the omega shape has been detected, and based on a determination that the omega shape has not been detected, the first processor 150 may transmit, to the second display device 200, the environment guide information for guiding adjustment of a posture and location of the patient.

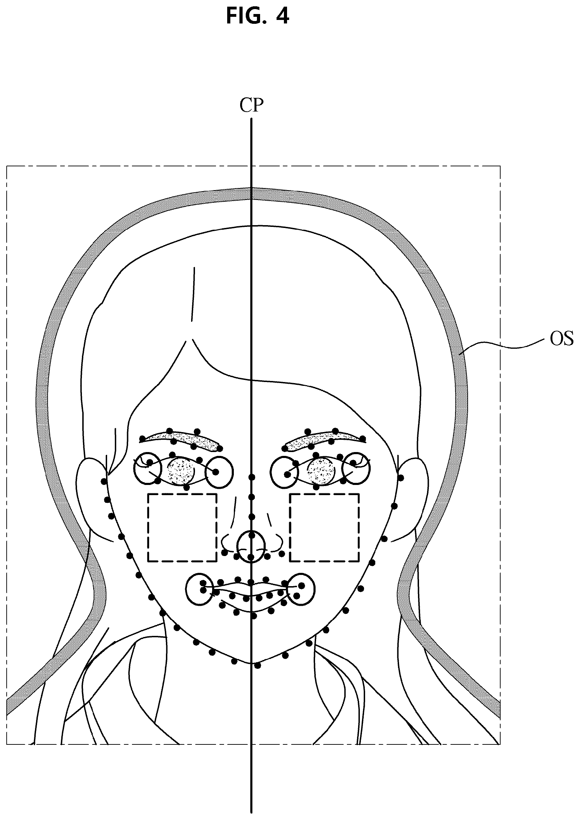

As shown in FIG. 4, detecting the omega shape (Ω-Shape) refers to detecting an outline from a top of the patient's head (i.e., crown) to a shoulder line in the patient's face image.

The first processor 150 may identify or otherwise set a line to a nose tip from the center (crown) of the patient's head recognized in the image as a center pole, and may divide the face image into the left face image and the right face image based on the set center pole.

The first processor 150 may also set a line from the center of the patient's head (i.e., the crown) to a center of the chin as the center pole.

The center of the patient's head is a center of the omega shape, which may be a point where a differential value is 0.

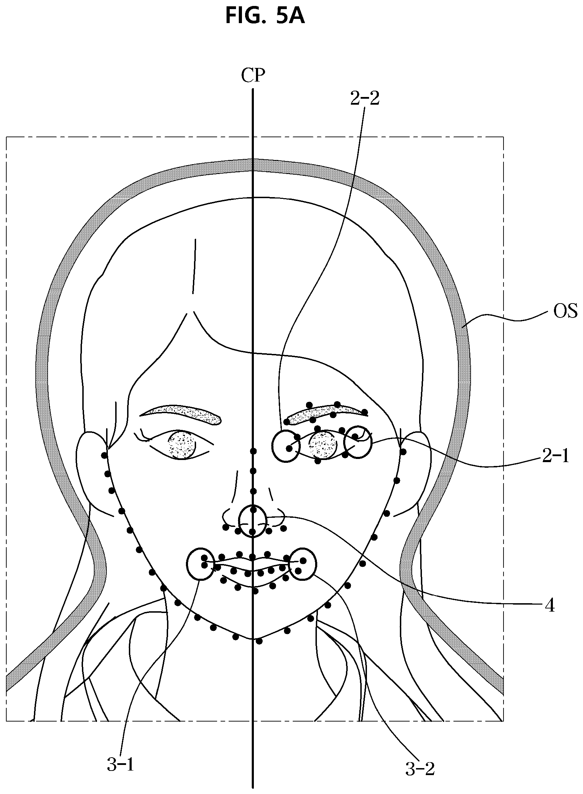

As shown in FIG. 5A, based on a determination that the omega shape has been detected, the first processor 150 may divide the face into left and right face images, and determine whether all feature points of the divided left and right face images exist. In this instance, the first processor 150 may determine whether at least one feature point of the left feature points of the left face image or the right feature points of the right face image has not been detected.

The first processor 150 may confirm position information of feature points of a face image on a side where all feature points are detected among the left and right face images, and may map feature points to a face image on a side where at least one feature point is not detected (i.e., where there is a missing feature point), based on the confirmed position information of the feature points and the center pole.

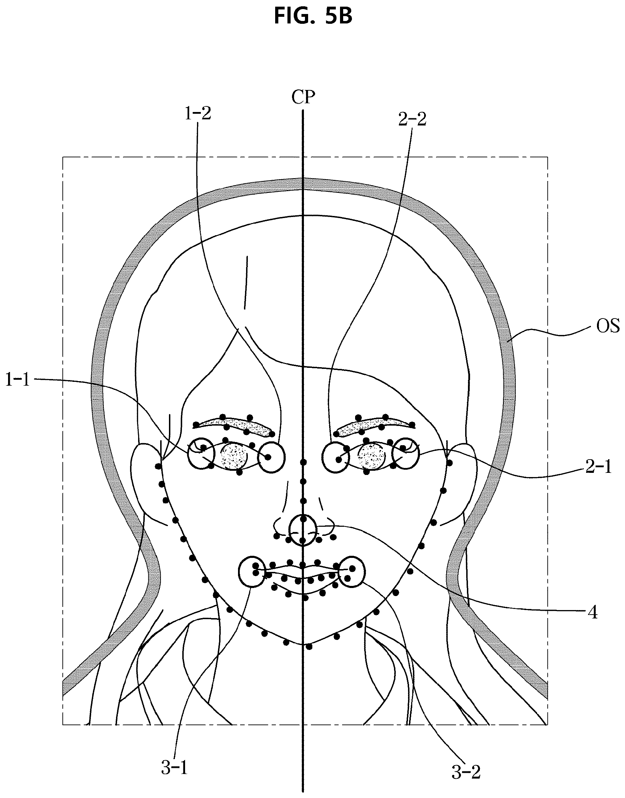

As shown in FIG. 5B, based on a determination that at least one feature point of the left feature points of the left face image has not been detected, the first processor 150 may confirm the right feature points of the right face image corresponding to a face landmark for which no feature point is detected (i.e., where there is a missing feature point), and may map the right feature points of the right face image (i.e., the detected feature points) to the left face based on the set center pole. That is, the first processor 150 may map the confirmed right feature points of the right face image to the left face image based on the center pole.

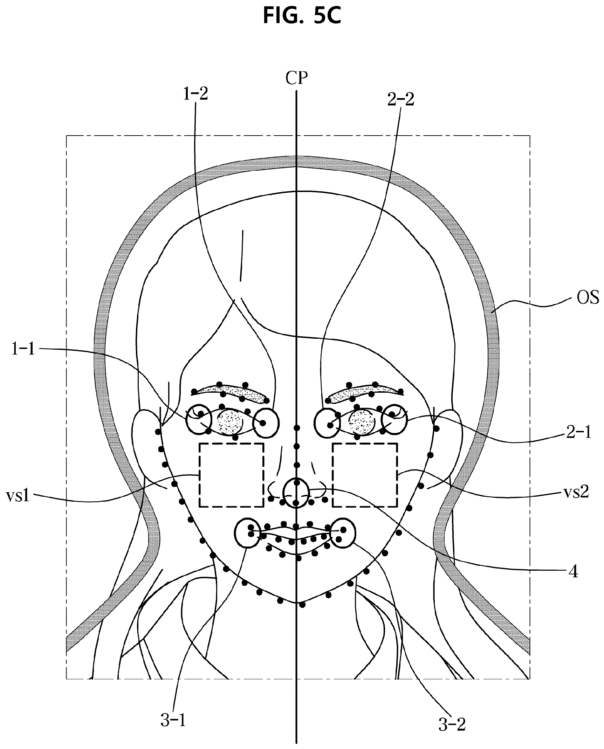

As shown in FIG. 5C, in response to the detection of the omega shape and the mapping of the feature points of the left and right face images, the first processor 150 may generate feature points of the left and right face images in the image, and may identify the vital sign detection areas vs1 and vs2 based on the generated feature points of the left and right face images.

Generating the feature points of the left and right face images in the image refers to generating feature points that are not detected from the image received from the second display device.

The first processor 150 may confirm vital signs in the identified vital sign detection areas and determine a health state of the patient based on the confirmed vital signs.

The first processor 150 may diagnose the patient's state based on the confirmed vital signs, control the first display 160 to display diagnosis result information, and transmit the diagnosis result information to the second display device.

The vital sign detection area may be an area selected by a user (i.e., doctor).

The vital sign detection area may be a diseased area.

**In** a case where the body is a face, the vital sign detection area may include an orbital area and an infraorbital area under the eyes in the left and right face images.

**In** a case where the body is a face, the vital sign detection area may include a pupil.

The first processor 150 may also determine the patient's state, i.e., an improved state or a deteriorated state, based on the diagnosis result information and diagnosis history information.

The first processor 150 may determine whether an environment of the second space is a low-light environment or an extremely low-light environment based on the illuminance information received from the second display device and the first and second reference illuminance information.

That is, the first processor 150 may determine whether the illuminance of the second space is less than the first reference illuminance based on the illuminance information received from the second display device 200, and may determine that the environment of the second space is the low-light environment based on a determination that the illuminance of the second space is less than the first reference illuminance.

The first processor 150 may determine whether the illuminance of the second space is less than the first reference illuminance and greater than or equal to the second reference illuminance based on the illuminance information received from the second display device 200, and may determine that the environment of the second space is the low-light environment based on a determination that the illuminance of the second space is less than the first reference illuminance and greater than or equal to the second reference illuminance.

The first processor 150 may determine whether the illuminance of the second space is less than the second reference illuminance based on the illuminance information received from the second display device 200. Based on a determination that the illuminance of the second space is less than the second reference illuminance, the first processor 150 may determine that the environment of the second space is an extremely low-light environment and transmit environment guide information for guiding adjustment of a brightness of the second lighting to the second display device 200. The second reference illuminance may be lower than the first reference illuminance.

In addition, the first processor 150 may obtain brightness information of the image based on the image information received from the second display device 200, and may determine whether the environment of the second space is a low-light environment based on the obtained brightness information.

The first processor 150 may also obtain brightness information between a body area and a background area in the image received from the second display device 200, and may determine whether the image is obtained in a backlight environment based on the obtained brightness information.

Based on a determination that the patient's image has been obtained in a backlight environment or a low-light environment, the first processor 150 may perform pre-processing to improve a luminance of the face area in the image using non-uniform illuminance image improvement technology or low-light image enhancement technology.

Based on a determination that the environment of the second space is a low-light environment, the first processor 150 may transmit, to the second display device 200, environment guide information for guiding the brightness adjustment of the second display 260 or the second lighting 290.

Based on a determination that the environment of the second space is a low-light environment or a backlight environment, the first processor 150 may control the brightness adjustment of the first display 160 or perform image pre-processing for non-uniform illuminance.

To adjust the brightness of the first display 160, the first processor 150 may control a current or voltage of the first display 160.

Based on a determination that the environment of the second space is a low-light environment and a backlight environment, the first processor 150 may transmit, to the second display device 200, environment guide information for guiding location adjustment of the second lighting 290 or the second image sensor 220.

Based on a determination that the omega shape has not been detected and the feature points of the left and right face images have not been detected, the first processor 150 may determine that patient recognition has failed, and may transmit, to the second display device 200, environment guide information for guiding adjustment of the patient's location and posture.

Based on a determination that the environment of the second space is a low-light environment, the omega shape has been detected, and the feature points of the left and right face images have not been detected, the first processor 150 may determine that the environment of the second space is a low-light backlight environment and may transmit, to the second display device 200, environment guide information for guiding the brightness adjustment of the second lighting in the second space.

Based on a determination that the environment of the second space is a low-light environment, the omega shape has been detected, and feature points of only one side (a first side) among the feature points of the left and right face images have all been detected, the first processor 150 may correct a brightness of the face image, may generate feature points of a second side of the face image based on the corrected image information, the feature points of face image of the first side, and information about the center pole of the omega shape, and then may identify the vital sign detection areas based on the feature points of the left and right face images.

Based on a determination that the illuminance of the second space is greater than or equal to the first reference illuminance, the omega shape has been detected, and the feature points of the left and right face images have not been detected, the first processor 150 may determine that the environment of the second space is a backlight environment, may correct a brightness of the face image, may re-detect the feature points of the left and right face images based on the corrected image information, and may identify the vital sign detection areas based on the re-detected feature points of the left and right face images.

That is, based on a determination that the illuminance of the second space is greater than or equal to the first reference illuminance and the environment of the second space is a backlight environment, the first processor 150 may perform pre-processing to improve a luminance of the face area in the image using non-uniform illuminance image improvement technology or low-light image enhancement technology.

Based on a determination that the illuminance of the second space is greater than or equal to the first reference illuminance, the omega shape has been detected, and feature points of face image of only one side (the first side) among the feature points of the left and right face images have all been detected, the first processor 150 may generate feature points of face image of the second side based on the feature points of the first side's face image and the information about the center pole of the omega shape, and then may identify the vital sign detection areas based on the feature points of the left and right face images.

The first processor 150 may obtain, from the second display device, a skin reflection coefficient of the patient based on first image information in an illuminated environment and second image information in an unilluminated environment, and may transmit required illuminance information corresponding to the obtained skin reflection coefficient to the second display device.

The illuminated environment includes an environment in which at least one of the second lighting or the second display 260 in the second space is turned on, and the unilluminated environment is an environment in which the second lighting and the second display 260 in the second space are turned off.

The required illuminance information transmitted to the second display device may include illuminance information of the second display 260, and may include illuminance information of the second display 260 and the second lighting.

The first processor 150 may transmit voice information received through the first microphone 170 to the second display device 200, and may control the first speaker 180 to output the voice information received from the second display device 200.

The first memory 151 may store the first and second reference illuminance information.

The first memory 151 may store the patient identification information, the reference biometric information, the medical care information and the diagnosis result information of the patient.

The patient's diagnosis result information may be stored in the first memory 151 as the patient's diagnosis history information by date.

The first memory 151 may further store diagnostic criteria information for each type of disease for diagnosis.

The diagnostic criteria information may include color information, pulse information, and spasm information for each type of disease, and may include position information and curvature information of feature points for each body part.

The first memory 151 and the first processor 150 may be implemented as separate chips. Alternatively, the first memory 151 and the first processor 150 may be implemented as a single chip.

The first display 160 may output information corresponding to a control command of the first processor 150 as an image.

The first display 160 may display an image with a display parameter corresponding to the control command of the first processor 150.

The first display 160 may display the patient's image and the patient's diagnosis history information. The patient's image may include an image of the patient's body. The patient's image may include an image of the patient's face.

The first display 160 may display a user input in response to the control command of the first processor 150.

The first display 160 may also display an image of a user. The user's image may include an image of a doctor.

The first display 160 may adjust a brightness of the patient's image and display a corrected image for the patient's image with non-uniform illuminance.

The first display 160 may display a vital sign detection area in the patient's image and display diagnosis result information.

The first display 160 may display an image corresponding to a user command. For example, an image corresponding to a user command may include a document image, a photo image, or a video in which medical information is recorded.

The first display 160 may be a Cathode Ray Tube (CRT), a Digital Light Processing (DLP) panel, a Plasma Display Panel, a Liquid Crystal Display (LCD) panel, an Electro Luminescence (EL) panel, Electrophoretic Display (EPD) panel, Electrochromic Display (ECD) panel, Light Emitting Diode (LED) panel, or Organic Light Emitting Diode (OLED) panel, etc., without being limited thereto.

The first display 160 may include a Touch Screen Panel (TSP) that forms a mutual layer structure with a touch pad.

The first microphone 170 may receive a user's voice and transmit voice information about the received voice to the first processor 150.

The first microphone 170 and the first image sensor 120 may be included in the first input interface 110.

The first speaker 180 may output sound information according to the control command of the first processor 150. The sound information may include voice information of the patient.

The first lighting 190 may be disposed around the first image sensor 120.

The first lighting 190 may emit light in response to a control command from the first processor 150.

The first lighting 190 may emit light with a brightness corresponding to the control command of the first processor 150.

The first lighting 190 may be physically separate from the first display 160.

The first lighting 190 may be a backlight unit provided on the first display 160.

At least one constituent component may be added or omitted corresponding to the performance of the constituent components of the first display device shown in FIG. 2. Also, it will be easily understood by those skilled in the art that mutual positions of the constituent components may be modified corresponding to the performance or structure of the first display device.

Each of the constituent components shown in FIG. 2 refers to software, and/or a hardware component such as Field Programmable Gate Array (FPGA) or Application Specific Integrated Circuit (ASIC).

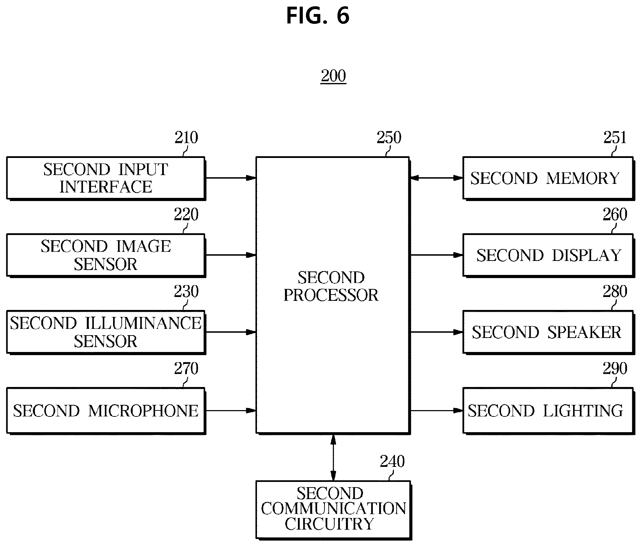

FIG. 6 is a control block diagram of a second display device according to an embodiment.

The second display device 200 may include a second input interface 210, a second image sensor 220, a second illuminance sensor 230, a second communication circuitry 240, a second processor 250, a second memory 251, and a second display 260, and may further include a second microphone 270, a second speaker 280, and a second lighting 290.

The second input interface 210 receives a user input and transmits the received user input to the second processor 250.

The user input may further include a power-on command and a power-off command.

The user input may include an on command and an off command for non-face-to-face medical care.

The second input interface 210 may receive patient identification information.

The second input interface 210 may receive an on command, an off command, and a sensing command of the second image sensor 220.

The sensing command of the second image sensor 220 may be a command to capture a patient.

The second input interface 210 may receive an on command and an off command of the second lighting 290.

The second input interface 210 may be physically separate from the second display 260.

The second input interface 210 may include a hardware device such as various buttons, switches, a pedal, a keyboard, a mouse, a track-ball, various levers, handles, and sticks.

In addition, the second input interface 210 may also include a Graphical User Interface (GUI) such as a touch pad, i.e., a software device. The touch pad is implemented as a Touch Screen Panel (TSP) and may form a mutual layer structure with the display.

The second image sensor 220 may obtain an image of a person around the second display device 200, convert image information about the obtained image into an electrical image signal, and transmit the converted image signal to the second processor 250.

In obtaining the image of the person, the second image sensor 220 may also obtain an image of a color patch provided by a medical institution.

That is, when an image of a patient's body is captured using the second image sensor 220, the patient's body may be photographed together with the color patch placed around the patient.

The second image sensor 220 may include a Charge-Coupled Device (CCD) image sensor or Complementary Metal Oxide Semiconductor (CMOS) image sensor.

The second image sensor 220 may include a camera. The second image sensor 220 may include a three-dimensional (3D) space recognition sensor such as a time-of-flight (TOF) camera, stereo camera, and the like.

The second illuminance sensor 230 may detect an illuminance around the second image sensor 220, and transmit illuminance information about the detected illuminance to the second processor 250.

The second image sensor 220 and the second illuminance sensor 230 may be physically separate from the second display 260.

The second communication circuitry 240 may include at least one constituent component enabling communication between components of the second display device 200.

The second communication circuitry 240 may include at least one constituent component enabling communication with an external device, for example, at least one of a short-range communication module, a wired communication module, or a wireless communication module. Here, the external device may include the first display device 100.

The second communication circuitry 240 may include both a wired network and a wireless network.

The second processor 250 oversees control related to operations of the second display device 200. A single or a plurality of second processors 250 may be provided. That is, at least one second processor 250 may be provided.

The second processor 250 performs control related to operations of the second display device 200 using data stored in the second memory 251.

**In** response to receiving medical care request information through the second input interface 210, the second processor 250 may transmit the received medical care request information to the first display device 100.

**In** response to receiving identification information of a medical institution and identification information of a doctor through the second input interface 210, the second processor 250 may transmit the medical care request information based on the received medical institution's identification information and the doctor's identification information.

**In** response to receiving a medical care approval command and guide information for non-face-to-face medical care from the first display device 100, the second processor 250 may control the second display 260 to display the received guide information. The second processor 250 may also control the second speaker to output the guide information.

The second processor 250 may transmit the image information received from the second image sensor 220 to the first display device 100.

The second processor 250 may transmit the illuminance information received from the second illuminance sensor 230 to the first display device 100.

The second processor 250 may control a brightness of the second lighting 290 in response to a control command received from the first display device 100.

The second processor 250 may control the second display 260 to display a corrected image in response to a control command received from the first display device 100.

The corrected image may be an image in which a brightness, uneven illuminance, or color of the image obtained by the second image sensor 220 has been corrected.

The second processor 250 may also control a brightness of the second display 260 in response to a control command received from the first display device 100.

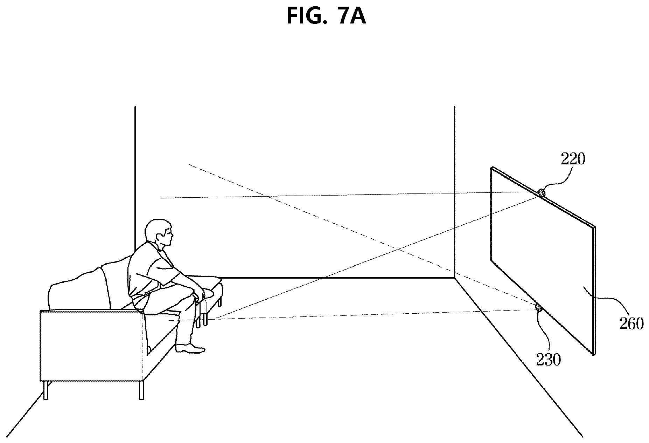

As shown in FIG. 7A, the second processor 250 may control the second display 260 to be turned on and control the second image sensor to obtain a first image of the patient while the second display 260 is turned on. The second processor 250 may obtain first illuminance information of a second space detected by the second illuminance sensor 230 when the first image is obtained. In this case, the second processor 250 may obtain the first image in an environment of external illuminance and lighting of the second screen.

Controlling to turn on the second display 260 refers to turning on a backlight unit provided on the second display 260.

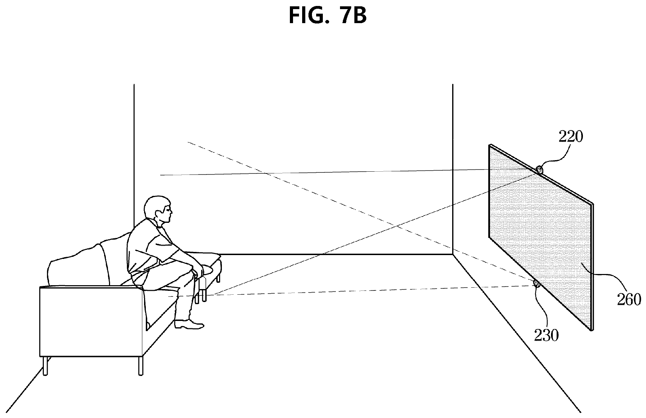

As shown in FIG. 7B, the second processor 250 may control the second display 260 to be turned off and control the second image sensor 220 to obtain a second image of the patient while the second display 260 is turned off. The second processor 250 may obtain second illuminance information of the second space detected by the second illuminance sensor 230 when the second image is obtained. In this case, the second processor 250 may obtain the second image in an environment where only external illuminance exists.

Controlling to turn off the second display 260 refers to turning off the backlight unit provided on the second display 260.

In a case where the second lighting exists in the second space, the second processor 250 may control the second display 260 and the second lighting to be turned on, and may control the operation of the second image sensor to obtain the first image of the patient with the second display 260 and the second lighting turned on. Also, the second processor 250 may control the second display 260 and the second lighting to be turned off, and may control the operation of the second image sensor 220 to obtain the second image of the patient with the second display 260 and the second lighting turned off.

Controlling to turn on the second display 260 and the second lighting refers to turning on the second display 260 and the second lighting. Also, controlling to turn off the second display 260 and the second lighting refers to turning off the second display 260 and the second lighting.

The second processor 250 may obtain brightness information of the first image and brightness information of the second image.

The second processor 250 may obtain average brightness information of a face area in the first image and average brightness information of a face area in the second image.

The second processor 250 may obtain a skin reflection coefficient of the patient based on the average brightness information F1 of the first image, the average brightness information F2 of the second image, first illuminance information Lux1, and second illuminance information Lux2. The second processor 250 may obtain required illuminance amount required by the second display 260 based on the obtained patient's skin reflection coefficient, and may control the brightness of the second display 260 or the brightness of the second lighting based on the obtained required illuminance amount.

The second processor 250 may control the brightness of the second display 260 in response to the obtained patient's skin reflection coefficient being less than a reference skin reflection coefficient.

In response to the obtained patient's skin reflection coefficient being less than the reference skin reflection coefficient, the second processor 250 may also control the brightness of the second display 260 based on a difference between the obtained patient's skin reflection coefficient and the reference skin reflection coefficient.

The required illuminance amount required by the second display device may include the amount of illuminance of the second display 260.

The average brightness information of the face area in the first image may be obtained, and the average brightness information of the face area in the second image may be obtained.

The second processor 250 may obtain the amount of change in average brightness of the face area in the image (F1-F2), the amount of change in illuminance sensor value (Lux1-Lux2), the illuminance change ratio (Lux1/Lux2), and an average brightness change ratio (F1/F2) of the face area.

The second processor 250 may determine that a skin tone of the patient is a darker skin tone, when the average brightness change ratio (F1/F2) of the face area to the illuminance change ratio (Lux1/Lux2) is significantly smaller.

The second processor 250 may obtain the skin reflection coefficient (R=F1/F2), obtain the required illuminance amount based on the obtained skin reflection coefficient, and control the brightness of the second display 260 based on the obtained required illuminance amount.
The required illuminance amount (L_a-L_c)=((Max-Min)*R)*α, (maximum compensation illuminance amount when α=1.0)
Current illuminance amount : L_c
Operational illuminance amount of the second display of the second display device : L_a
Max : maximum compensation illuminance amount
Min : minimum compensation illuminance amount

The second processor 250 may obtain image information about the color patch provided by the medical institution and transmit the obtained image information of the color patch to the first display device 100.

The image obtained by the second image sensor may include both the image of the patient's body and the image of the color patch.

The color patch provided by the medical institution may be a printout including multiple colors, or an image file transmitted to a user device of the patient.

The second processor 250 may control the second display 260 to display the corrected image received from the first display device 100.

The second processor 250 may control the second display 260 to display environment guide information received from the first display device 100.

The second processor 250 may also control the second display 260 based on a display parameter received from the first display device 100.

The second processor 250 may transmit, to the first display device 100, image information re-obtained by the second image sensor 220 after displaying the environment guide information. In this case, the second processor 250 may also transmit illuminance information detected by the second illuminance sensor to the first display device 100.

The second processor 250 may display diagnosis result information received from the first display device 100.

The second processor 250 may transmit voice information received through the second microphone 270 to the first display device 100, and control the second speaker 280 to output the voice information received from the first display device 100.

The second memory 251 may store identification information of a user (i.e., the patient).

The second memory 251 may store identification information of a medical institution, identification information of a doctor, and identification information of the first display device.

The identification information of the first display device may be identification information of the first display device located in a medical institution where face-to-face medial care has been registered.

The second memory 251 may store brightness information of the second display 260 to obtain an image of patient in an optimal environment, and may store brightness information of the second lighting.

The brightness information may include illuminance information.

The second memory 251 may store diagnosis result information.

The second memory 251 and the second processor 250 may be implemented as separate chips. Alternatively, the second memory 251 and the second processor 250 may be implemented as a single chip.

The second display 260 may output information corresponding to a control command of the second processor 250 as an image.

The second display 260 may display an image of the patient. The patient's image may include an image of the patient's body. The patient's image may include an image of the patient's face.

The second display 260 may also display an image of a user (i.e., a doctor) using the first display device 100.

The second display 260 may display a user input in response to the control command of the second processor 250.

The second display 260 may display an image according to a display parameter corresponding to the control command of the second processor 250.

The second display 260 may be a Cathode Ray Tube (CRT), a Digital Light Processing (DLP) panel, a Plasma Display Panel, a Liquid Crystal Display (LCD) panel, an Electro Luminescence (EL) panel, Electrophoretic Display (EPD) panel, Electrochromic Display (ECD) panel, Light Emitting Diode (LED) panel, or Organic Light Emitting Diode (OLED) panel, etc., without being limited thereto.

The second display 260 may include a Touch Screen Panel (TSP) that forms a mutual layer structure with a touch pad.

The second microphone 270 may receive a user's voice and transmit voice information about the received voice to the second processor 250.

The second microphone 270 and the second image sensor 220 may be included in the second input interface 210.

The second speaker 280 may output sound information according to the control command of the second processor 250. The sound information may include voice information of a doctor.

The second lighting 290 may be disposed around the second image sensor 220.

The second lighting 290 may emit light in response to a control command from the second processor 250. The second lighting 290 may adjust a brightness of the light emitted in response to the control command of the second processor 250.

The second lighting 290 may be physically separate from the second display 260.

The second lighting 290 may include a backlight unit provided on the second display 260.

At least one constituent component may be added or omitted corresponding to the performance of the constituent components of the second display device shown in FIG. 6. Also, it will be easily understood by those skilled in the art that mutual positions of the constituent components may be modified corresponding to the performance or structure of the second display device.

Each of the constituent components shown in FIG. 6 refers to software, and/or a hardware component such as Field Programmable Gate Array (FPGA) or Application Specific Integrated Circuit (ASIC).

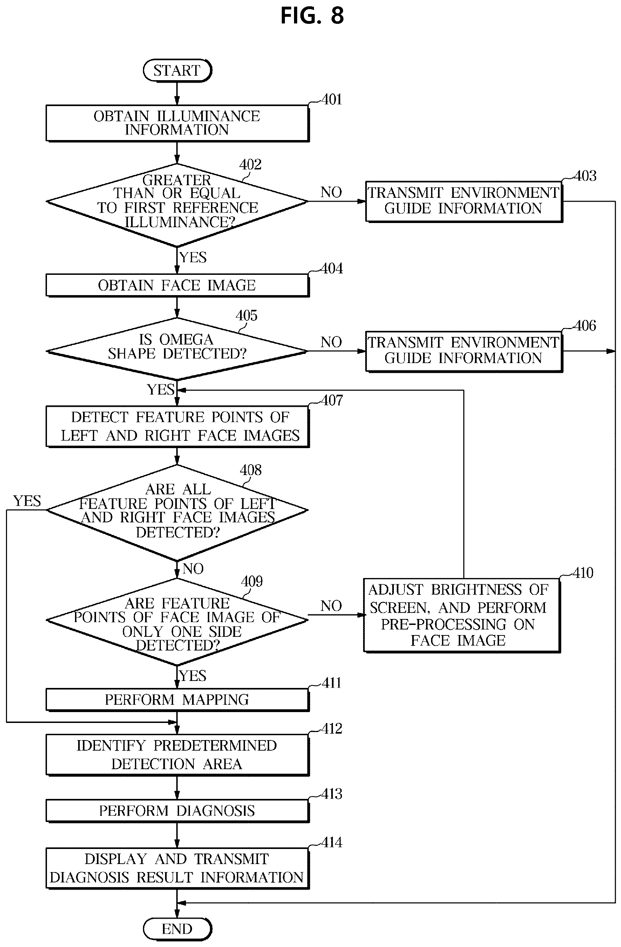

FIG. 8 is a flowchart of a method for controlling a first display device according to an embodiment.

In response to receiving medical care request information and patient identification information from the second display device 200, the first display device may display the received medical care request information and patient identification information on the first display 160.

In response to receiving a medical care approval command through the first input interface 110, the first display device may transmit the received medical care approval command and guide information for non-face-to-face medical care to the second display device 200.

In response to receiving image information of a patient from the second display device 200, the first display device may display the received patient's image information on the first display 160.

In displaying the patient's image information on the first display 160, the first display device may identify image information of a color patch from the patient's image information received from the second display device 200, and may obtain color information of the color patch based on the identified image information of the color patch. Also, the first display device may obtain color correction information based on the obtained color information of the color patch and pre-stored color information of color patch, may correct the patient's image information based on the obtained color correction information, and may display the corrected image information.

The first display device may transmit the corrected image information to the second display device 200. **In** this case, the second display device 200 may display the corrected image information on the second display 260.

The first display device may obtain illuminance information transmitted from the second display device 200 (401), and determine whether an illuminance of a second space is greater than or equal to a first reference illuminance based on the obtained illuminance information and first reference illuminance information (402). Here, the second space may be a location where the patient is located and remote non-face-to-face medical care is performed.

Based on a determination that the illuminance of the second space is less than the first reference illuminance, the first display device may transmit, to the second display device, environment guide information for guiding adjustment of a brightness of the second lighting 290 (403). That is, the first display device may transmit, to the second display device, environment guide information that guides the second lighting 290 to be turned on or environment guide information that guides brightness adjustment of the second lighting to increase the brightness of the second lighting.

The first display device may control a brightness of the first display to be increased.

Based on a determination that the illuminance of the second space is less than the first reference illuminance, the first display device may also transmit, to the second display device 200, environment guide information that guides brightness adjustment of the second display 260.

In response to re-receiving a face image from the second display device in response to the transmission of the environment guide information, the first display device may recognize a vital sign detection area based on the re-received face image. In this case as well, the first display device may obtain illuminance information transmitted from the second display device 200, and may determine whether the illuminance of the second space is greater than or equal to the first reference illuminance based on the obtained illuminance information and the first reference illuminance information.

Based on a determination that the illuminance of the second space is greater than or equal to the first reference illuminance, the first display device may obtain a face image by recognizing the face based on the image information (404).

The first display device may detect an omega shape based on the obtained face image. In this instance, the first display device may determine whether the omega shape has been detected from the face image (405).

Based on a determination that the omega shape has not been detected, the first display device may transmit, to the second display device, environment guide information that guides adjustment of a posture and a location of the patient (406). In this case, the second display device may display environment guide information that guides the patient to face the second image sensor for photographing a body or face of the patient.

Based on a determination that the omega shape has been detected, the first display device may detect feature points of the face image (407).

Based on a determination that the feature points of the face image have been detected, the first display device may perform labeling on the detected feature points and obtain a nose tip point based on the labeled information.

The first display device may confirm a crown of head in the omega shape, and may set a center pole by connecting the confirmed crown and the nose tip point.

The first display device may distinguish a left face image and a right face image in the face image based on the set center pole, and may determine whether all feature points of the left face image and the right face image have been detected (408) based on pre-stored labeling information of feature points of the left face image, pre-stored labeling information of feature points of the right face image, labeling information of the detected feature points of the left face image, and labeling information of the detected feature points of the right face image.

Based on a determination that not all the feature points of the left face image and the right face image have been detected(409), the first display device may confirm a face of one side where not all the feature points have been detected among the left feature points of the left face image and the right feature points of the right face image.

Based on a determination that not all the feature points of the left face image and the right face image have been detected, the first display device may determine that an environment of the second space is a backlight environment, adjust a brightness of the first display, and perform pre-processing on the face image to improve non-uniform illuminance (410).

The first display device may re-detect feature points of the face image based on the processed face image.

Based on a determination that at least one of feature points of a face image of a first side among the left face image and the right face image has not been detected, the first display device may determine that the face in the face image is asymmetric. In this case, the first display device may map (411) feature points of a face image of a second side (i.e., the detected feature points) to the face image of the first side (i.e., the side corresponding to the missing feature points) based on the center pole of the omega shape, thereby generating all feature points of the face image of the first side.

The first display device may identify a predetermined detection area based on the feature points of the left and right face images (412). Here, the predetermined detection area may be an area for detecting a vital sign. That is, the first display device may identify the vital sign detection area from the left and right face images.

The first display device may recognize vital sign information in the vital sign detection area, and may diagnose a health state of the patient based on the recognized vital sign information and reference vital sign information (413).

The first display device may generate diagnosis result information about a diagnosis result, display the generated diagnosis result on the first display, and transmit the generated diagnosis result information to the second display device (414).

The first display device may also identify the vital sign detection area based on an face image received in a state where the illuminance of the second space is less than the first reference illuminance. A control process of the first display device in the above case is briefly described below.

**In** response to the illuminance of the second space being less than the first reference illuminance, the first display device may determine that the environment of the second space is a low-light environment.

The first display device may recognize a face based on the image information received from the second display device, thereby obtaining a face image.

The first display device may detect an omega shape and feature points of the face image based on the obtained face image. **In** this instance, the first display device may determine whether the omega shape has been detected from the face image.

Based on a determination that both the omega shape and the feature points of the face image have not been detected, the first display device may determine that the environment of the second space is a low-light backlight environment, and may transmit, to the second display device 200, environment guide information for guiding adjustment of a location of the second lighting 290 or a location of the second image sensor 220. **In** this instance, the second display device may display the environment guide information that guides the location adjustment of the second lighting 290 or the second image sensor 220 through the second display.

Based on a determination that the omega shape has been detected and all feature points of the face image of the first side among the feature points of the left and right face images have been detected, the first display device may correct a brightness of the face image, may generate feature points of the face image of the second side based on the corrected image information, the feature points of the face image of the first side and information about the center pole of the omega shape, and then may identify vital sign detection areas of the left and right face images based on the feature points of the first side's face image and the feature points of the second side's face image.

Based on a determination that both the omega shape and all the feature points of the left and right face images have been detected in a state where the illuminance of the second space is less than the first reference illuminance, the first display device may identify vital sign detection areas of the left and right face images based on the feature points of the left and right face images.

In response to the illuminance of the second space being less than the first reference illuminance, the first display device may determine whether the illuminance of the second space is less than a second reference illuminance. Based on a determination that the illuminance of the second space is less than the second reference illuminance, the first display device may determine that the environment of the second space is an extremely low-light environment, and may transmit, to the second display device 200, environment guide information for guiding brightness adjustment of the second lighting. The second reference illuminance may be lower than the first reference illuminance.

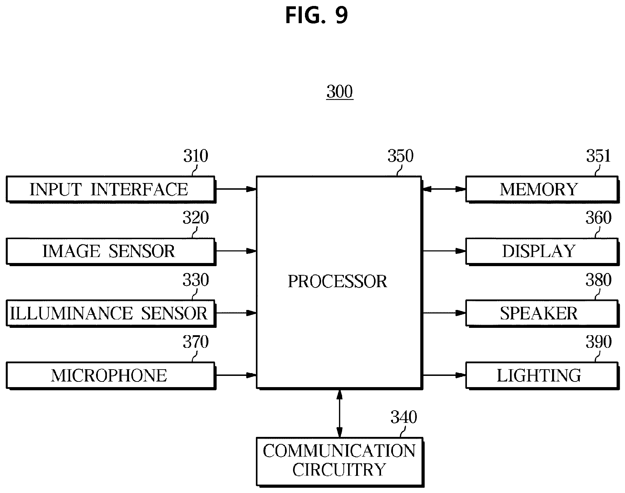

FIG. 9 is a control block diagram of a display device according to another embodiment
A display device 300 according to another embodiment may be a user device.

The user device may be carried by a user, or placed in a user's home or office. The user device may include a personal computer, a laptop, a tablet PC, a portable telephone, a smartphone, a handheld device, a wearable device, and the like, without being limited thereto.

The display device may execute an application of a medical diagnostic platform that provides services for checking, diagnosing, and managing a user's health state. The application may be an application program for providing medical diagnosis services.

The display device 300 may control a download, setup, and execution of the application for providing medical diagnosis services, and may provide a user with an image corresponding to the execution of the application.

The display device 300 may communicate with a server for medical diagnosis and management, a web server for a website, an application server that provides medical diagnosis services, and a database server.

The display device 300 may communicate with at least one of home appliances, massage devices, or home medical devices.

The display device 300 according to another embodiment may include an input interface 310, an image sensor 320, an illuminance sensor 330, a communication circuitry 340, a processor 350, a memory 351, and a display 360, and may further include a microphone 370, a speaker 380, and a lighting 390.

The input interface 310 receives a user input and transmits the received user input to the processor 350. Here, a user may be a patient.

The user input may include a first command to collect biometric information of a user in a healthy state, and a second command to collect biometric information of a user seeking medical care.

The input interface 310 may receive user identification information and a user registration command.

The input interface 310 may receive a sensing command from the image sensor 120.

The sensing command of the image sensor 320 may be either the first command or the second command.

The input interface 310 may receive an on command and an off command of the lighting 390.

The image sensor 320 may obtain an image of the user of the display device 300, convert image information about the obtained user's image into an electrical image signal, and transmit the converted image signal to the processor 350.

The illuminance sensor 330 may detect an illuminance around the image sensor 320 and transmit illuminance information about the detected illuminance to the processor 350.

The communication circuitry 340 may include at least one constituent component enabling communication between components of the display device 300.

The communication circuitry 340 may include at least one constituent component enabling communication with an external device, for example, at least one of a short-range communication module, a wired communication module, or a wireless communication module. Here, the external device may include a server of a medical institution and a medical information server.

The communication circuitry 340 may include both a wired network and a wireless network.

The processor 350 oversees control related to operations of the display device 300. A single or a plurality of processors 350 may be provided. That is, at least one processor 350 may be provided.

The processor 350 performs control related to operations of the display device 300 using data stored in the memory 351.

The processor 350 may control execution or termination of the application based on a user input received through the input interface 310.

**In** response to receiving a user registration command and user information from the input interface 310, the processor 350 may register user information for medical care and diagnosis based on the received user information.

The user information may be user identification information to identify a user, and may include a date of birth, a name, an address, contact information, and code information of the user.

**In** response to receiving the first command from the input interface 310, the processor 350 may control the image sensor 320 to be activated, may obtain reference biometric information of the user based on image information of the user obtained by the activated image sensor 320, and may control the memory 351 to store the obtained reference biometric information of the user.

The first command may be a command to obtain reference biometric information of a user in a healthy state.

The user's image obtained in response to receiving the first command may be an image of the user's body in a healthy state. The body may include at least one of the face, arms, legs, or torso.

The reference biometric information may include reference color information of body and reference vital sign information of a predetermined area. For example, a vital sign may include tremor due to pulse or tremor due to spasm.

In obtaining the user's reference biometric information, the processor 350 may control the lighting 390 based on preset environment information.

The preset environment may include an environment in which the lighting 390 is turned off, an environment in which the lighting 390 is turned on, or an environment with a preset illuminance in a space where the display device 300 is installed.

The processor 350 may control a brightness of the lighting 390 to allow the space to become an environment with the preset illuminance.

In obtaining the user's reference biometric information, the processor 350 may also control a brightness of the display 360 to allow an illuminance of the space to become the preset illuminance.

In response to receiving the second command from the input interface 310, the processor 350 may control the lighting 390 based on preset environment information, may control the image sensor 320 to be activated, and then may obtain biometric information for diagnosis based on image information of the user obtained by the activated image sensor 320. Also, the processor 350 may control the display 360 to display the obtained biometric information for diagnosis.

The processor 350 may control the display 360 to display the user identification information and diagnosis history information of the user along with the user's image information.

The processor 350 may obtain reference biometric information stored in the memory 351, may obtain diagnosis result information by comparing the biometric information for diagnosis with the reference biometric information, and may control the display 360 to display the obtained diagnosis result information.

The processor 350 may obtain the diagnosis result information based on diagnostic criteria information stored in a server or the memory 351.

The processor 350 may obtain the diagnosis result information based on the diagnostic criteria information and biometric information for diagnosis stored in the memory 351.

For example, the processor 350 may detect tremor information from a predetermined detection area, and may diagnose the user's health based on reference tremor information corresponding to the diagnostic criteria information and the detected tremor information.

**In** displaying the user's image information on the display 360, the processor 350 may adjust a display parameter of at least one of the image or the display 360.

For example, the display parameter may include a brightness, saturation, contrast, and color of the display 360, and may include a brightness, saturation, contrast, and color of the image.

The processor 350 may identify image information of a color patch from the user's image information, and may obtain color information of the color patch based on the identified image information of the color patch. The processor 350 may obtain color correction information based on the obtained color information of the color patch and pre-stored color information of color patch, and may correct the user's image information based on the obtained color correction information.

In addition, the processor 350 may change an illuminance of the space based on a user's skin tone, before obtaining the user's image information for obtaining the reference biometric information or before obtaining the user's image information for diagnosis.

More specifically, the processor 350 may control the display 360 to be turned on, may control an operation of the image sensor 320 to obtain a first image of the user while the display 360 is turned on, and may obtain first illuminance information of the space detected by the illuminance sensor 330 upon obtaining the first image.

The processor 350 may control the display 360 to be turned off, control an operation of the image sensor 320 to obtain a second image of the user while the display 360 is turned off, and obtain second illuminance information of the space detected by the illuminance sensor 330 upon obtaining the second image.

The processor 350 may obtain average brightness information of a face area in the first image and obtain average brightness information of a face area in the second image.

The processor 350 may obtain a skin reflection coefficient of the user based on the average brightness information F1 of the first image, the average brightness information F2 of the second image, first illuminance information Lux1, and second illuminance information Lux2. Also, the processor 350 may obtain required illuminance amount required for diagnosis based on the obtained user's skin reflection coefficient, and may control at least one of the illuminance of the display 360 or the illuminance of the lighting 390 based on the obtained required illuminance amount.

The processor 350 may determine whether the illuminance of the image is uniform based on the user's image information, and based on a determination that the illuminance of the image is non-uniform, the processor 350 may correct the received user's image information to obtain an image with uniform illuminance.

The processor 350 may confirm illuminance information detected by the illuminance sensor 330 and correct a brightness of the received user's image information based on the confirmed illuminance information.

A configuration for image correction and a configuration for outputting environment guide information in a low-light environment, an extremely low-light environment, and a backlight environment are the same as those of the first processor, and thus description thereof is omitted herein.

The processor 350 may detect body feature points based on the user's image information and perform diagnosis based on the detected body feature points.

The processor 350 may detect the body feature points and an omega shape based on the user's image information, and may identify a vital sign detection area based on whether the body feature points have been detected, whether the omega shape has been detected, position information of the detected body feature points, and the center pole of the omega shape. The user's image information may be two-dimensional (2D) image information or three-dimensional (3D) image information.

The processor 350 may recognize a face based on the user's image information and detect feature points and an omega shape from the recognized face. Also, the processor 350 may obtain biometric information in the face based on whether feature points of the face image have been detected, whether the omega shape has been detected, position information of the detected feature points of the face image, and the center pole of the omega shape.

The processor 350 may identify a vital sign detection area using the biometric information. A configuration for identifying the vital sign detection area in the face is the same as that of the first processor, and thus a description thereof is omitted herein.

In response to receiving an aesthetic mode through the input interface 310, the processor 350 may recognize a face based on the user's image information, may detect feature points and an omega shape from the recognized face, and may distinguish a left face image and a right face image based on the detected omega shape. Also, the processor 350 may determine whether a cosmetic procedure or plastic surgery is required based on color information of the left and right face images, position information of feature points of the left and right face images, and information about distribution of the feature points of the left and right face images, and may control the display 360 to display suggestion information based on a determination that the cosmetic procedure or plastic surgery is required.

The processor 350 may display the left and right face images in which analysis information is matched, and may control the display 360 to display the suggestion information for cosmetic procedure or plastic surgery for each facial part.

In response to receiving the aesthetic mode through the input interface 310, the processor 350 may obtain reference biometric information of the face image stored in the memory 351, may obtain change information in the face image over time by comparing the obtained reference biometric information of the face image with currently detected biometric information of face image, and may determine whether a cosmetic procedure or plastic surgery is required based on the change information in the face image.

In response to receiving a makeup mode through the input interface 310, the processor 350 may recognize the face based on the user's image information, may detect the feature points and omega shape from the recognized face, and may distinguish a left face image and a right face image based on the detected omega shape. Also, the processor 350 may control the display 360 to display makeup suggestion information based on color information of the left and right face images, position information of feature points of the left and right face images, and information about distribution of the feature points of the left and right face images.

In response to receiving a body shape correction mode through the input interface 310, the processor 350 may control the display 360 to display guide information for guiding a posture or movement to be taken by a user to analyze a body shape of the user, and may control the speaker 380 to output the guide information as a voice.

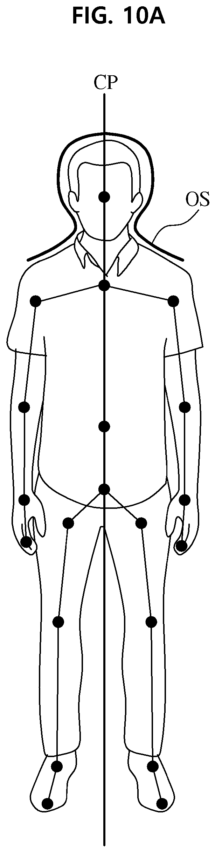

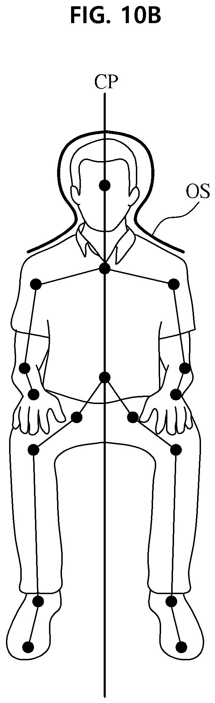

As shown in FIG. 10A and FIG. 10B, the processor 350 may guide the user to obtain an image after assuming a standing or sitting posture, or after assuming a walking posture.

As shown in FIG. 10, the processor 350 may recognize the entire body based on the user's image information, may detect the feature points and the omega shape from the recognized body, may set the center pole based on the detected omega shape, and may determine whether the body is balanced based on the set center pole and the body feature points.

Based on a determination that the user's body is unbalanced, the processor 350 may obtain front and rear position information, left and right position information, and up and down position information of each part of the user's body based on position information of the feature points of each body part, distribution information of the feature points, and tilt information (balance information) of the body distinguished by the omega shape. Also, the processor 350 may confirm a type and method of exercise required for the user based on the obtained front and rear position information, left and right position information, and up and down position information of each body part, and may control the display 360 to display the confirmed type and method of exercise.

For example, the processor 350 may guide a shoulder exercise method in response to a left-right asymmetry of the shoulder or a front-to-back asymmetry of the shoulder based on the center pole of the omega shape and a shoulder line, and may guide a pelvis exercise method in response to a left-right asymmetry of the pelvis based on the center pole of the omega shape and a pelvis line.

The body feature points may include feature points corresponding to a skeleton.

In response to receiving a massage mode through the input interface 310, the processor 350 may obtain a massage mode required for the user based on the obtained front and rear position information, left and right position information, and up and down position information of each body part, and may control the display 360 to display the massage mode.

Based on a determination that the processor 350 is able to communicate with a massage device or home medical device, the processor 350 may transmit information about the obtained massage mode to the massage device or home medical device.

The processor 350 may obtain a next diagnosis time based on diagnosis history information and diagnosis result information, and may control the display 360 to display information about the obtained next diagnosis time.

The processor 350 may recognize changes in user's health state based on the diagnosis history information and the diagnosis result information, and may control the display 360 to display the recognized change in the user's health state.

Information used for analyzing a face or body shape in the aesthetic mode, the makeup mode, and the body shape correction mode, or information suggested to the user according to each mode may be obtained and updated through Artificial Intelligence (AI) and learning.

The memory 351 may store the first and second reference illuminance information.

The memory 351 may store the user identification information, reference biometric information, medical care information and diagnosis result information of the user.

The memory 351 may further store diagnostic criteria information for each type of disease for diagnosis.

The diagnostic criteria information may include color information, pulse information, and spasm information for each type of disease, and may include position information and curvature information of feature points for each body part.

The memory 351 and the processor 350 may be implemented as separate chips. Alternatively, the memory 351 and processor 350 may be implemented as a single chip.

The display 360 may output information corresponding to a control command of the processor 350 as an image.

The display 360 may display a user's image and the user's diagnosis history information. The user's image may include an image of the user's body. The user's image may include an image of the user's face.

The display 360 may display a user input in response to a control command of the processor 350.

The display 360 may also display the user's image. The user's image may include an image of a doctor.

The display 360 may adjust a brightness of the user's image and display a corrected image for the user's image with non-uniform illuminance.

The display 360 may display a vital sign detection area in the user's image and display diagnosis result information.

The display 360 may display an image corresponding to a user command. For example, the image corresponding to the user command may include a document image, a photo image, or a video in which medical information is recorded.

The display 360 may include a Touch Screen Panel (TSP) that forms a mutual layer structure with a touch pad.

The microphone 370 may receive a user's voice and transmit voice information about the received voice to the processor 350.

The speaker 380 may output sound information according to the control command of the processor 350. The speaker 380 may output environment guide information as a voice.

The lighting 390 may be disposed around the image sensor 320.

The lighting 390 may emit light in response to the control command from the processor 350.

The lighting 390 may emit light with a brightness corresponding to the control command of the processor 350.

The lighting 390 may be a backlight unit provided on the display 360.

The AI-related functions according to the disclosure are operated through the processor 350 and the memory 351. The processor 350 may be a single or a plurality of processors. In this instance, the single or the plurality of processors 350 may be a general-purpose processor such as a Central Processing Unit (CPU), an Application Processor (AP), or Digital Signal Processor (DSP), a graphics-specific processor such as a Graphics Processing Unit (GPU) or Vision Processing Unit (VPU), or an AI-specific processor such as a Neural Processing Unit (NPU).

The single or the plurality of processors 350 may control input data to be processed according to predefined operation rules or AI models stored in the memory 351. Alternatively, in a case where the single or the plurality of processors may be AI-specific processors, the AI- specific processors may be designed with a hardware structure specialized for processing a specific AI model.

The predefined operation rules or AI models are characterized by being made through learning. Here, being made through learning refers to that a basic AI model is trained using a large amount of training data by a learning algorithm, thereby creating a predefined operation rule or AI model set to perform desired features (or desired objective). Such training may be performed in a device itself that performs the artificial intelligence according to the disclosure, or may be performed by a separate server and/or system. Examples of learning algorithms include supervised learning, unsupervised learning, semi-supervised learning, or reinforcement learning, without being limited thereto.

An AI model may include a plurality of neural network layers. Each of the plurality of neural network layers includes a plurality of weight values, and is configured to perform a neural network computation through a computation result of a previous layer and computation among the plurality of weight values. The plurality of weight values of the plurality of neural network layers may be optimized by a learning result of the AI model. For example, the plurality of weight values may be updated so that a loss value or a cost value obtained from the AI model is reduced or minimized during a learning process. The artificial neural networks may include a Deep Neural Network (DNN), Convolutional Neural Network (CNN), Recurrent Neural Network (RNN), Restricted Boltzmann Machine (RBM), Deep Belief Network (DBN), Bidirectional Recurrent Deep Neural Network (BRDNN), or Deep Q-Networks, or the like, but are not limited to the examples described above.

At least one component may be added or omitted in accordance with the performance of the components of the display device shown in FIG. 9. Also, it will be easily understood by those skilled in the art that mutual positions of the components may be modified corresponding to the performance or structure of the display device.

Each of the components shown in FIG. 9 refers to a software and/or hardware component such as a Field Programmable Gate Array (FPGA) and Application Specific Integrated Circuit (ASIC).

The disclosed embodiments may be implemented in the form of a recording medium that stores instructions executable by a computer. The instructions may be stored in the form of program codes, and when executed by a processor, the instructions may create a program module to perform operations of the disclosed embodiments. The recording medium may be implemented as a computer-readable recording medium.

The computer-readable recording medium may include all kinds of recording media storing instructions that may be interpreted by a computer. For example, the computer-readable recording medium may be a Read Only Memory (ROM), a Random Access Memory (RAM), a magnetic tape, a magnetic disk, a flash memory, an optical data storage device, etc.

Although embodiments of the disclosure have been described with reference to the accompanying drawings, a person having ordinary skilled in the art will appreciate that other specific modifications may be easily made without departing from the technical spirit or essential features of the disclosure. Therefore, the foregoing embodiments should be regarded as illustrative rather than limiting in all aspects.

## Claims

1. A display device comprising:
a display;
communication circuitry configured to communicate with an external device; and
at least one memory storing one or more instructions; and
at least one processor configured to execute the one or more instructions,
wherein the one or more instructions, when executed by the at least one processor, cause the display device to:
display an image on the display based on image information received through the communication circuitry,
identify, in the image, one or more feature points of a patient and an omega shape of the patient,
identify, based on the one or more feature points and the omega shape, whether the image includes a predetermined detection area associated with biometric information of the patient, and
based on identifying that the image includes the predetermined detection area, adjust a display parameter of at least one of the display or the image.

2. The display device of claim 1, wherein the one or more instructions, when executed by the at least one processor, further cause the display device to:
identify a center pole of the image,
identify, in the image, a left side of the patient and a right side of the patient based on the center pole, the omega shape and the one or more feature points,
identify whether a left feature point on the left side of the patient is included in the one or more feature points,
identify whether a right feature point on the right side of the patient is included in the one or more feature points, and
based on identifying that only one of the left feature point and the right feature point is included in the one or more feature points, map a feature point from among the left feature point and the right feature point that is included in the one or more feature points to a corresponding opposite side of the image.

3. The display device of claim 1, wherein the one or more instructions, when executed by the at least one processor, further cause the display device to:
based on identifying that at least one of the omega shape or the one or more feature points of the patient is not included in the image, control the communication circuitry to transmit, to the external device, environment guide information, and
wherein the environment guide information comprises information related to an adjustment of a location of the patient and a posture of the patient.

4. The display device of claim 1, wherein the one or more instructions, when executed by the at least one processor, further cause the display device to adjust the display parameter of the image based on illuminance information received through the communication circuitry and predetermined reference illuminance information.

5. The display device of claim 1, wherein the one or more instructions, when executed by the at least one processor, further cause the display device to control the communication circuitry to transmit environment guide information about illuminance adjustment to the external device, based on illuminance information received through the communication circuitry and predetermined reference illuminance information.

6. The display device of claim 1, wherein the one or more instructions, when executed by the at least one processor, further cause the display device to:
based on identifying that a brightness of the image is non-uniform, perform pre-processing on the image to make the brightness of the image uniform.

7. The display device of claim 1, wherein the at least one memory further stores reference biometric information of the patient, and
wherein the one or more instructions, when executed by the at least one processor, further cause the display device to, based on receiving medical care request information through the communication circuitry:
detect the biometric information of the patient from the predetermined detection area,
obtain diagnosis result information based on the detected biometric information and the stored reference biometric information, and
control the communication circuitry to transmit the obtained diagnosis result information to the external device.

8. The display device of claim 1, wherein the one or more instructions, when executed by the at least one processor, further cause the display device to:
based on identifying the omega shape and not identifying the one or more feature points, identify whether the image information was obtained in a backlit environment based on brightness information of the image information, and
based on identifying that the image information was obtained in the backlit environment, adjust the display parameter of the image and perform pre-processing on the image to make a brightness of the image uniform.

9. The display device of claim 8, wherein the one or more instructions, when executed by the at least one processor, further cause the display device to, based on identifying the omega shape and not identifying the one or more feature points:
obtain illuminance information from the external device through the communication circuitry,
identify whether an illuminance of a space where the external device is located is less than a first reference illuminance and greater than or equal to a second reference illuminance based on the illuminance information, first reference illuminance information and second reference illuminance information,
based on the illuminance of the space being less than the first reference illuminance and greater than or equal to the second reference illuminance, identify that the image information was obtained in the backlit environment, and
based on the illuminance of the space being less than the second reference illuminance, transmit environment guide information to the external device,
wherein the second reference illuminance is less than the first reference illuminance, and
wherein the environment guide information comprises information related to an adjustment to the illuminance of the space.

10. The display device of claim 1, wherein the one or more feature points comprises one or more feature points of a face of the patient,
wherein the one or more feature points of the face comprise a pair of left eye endpoints, a pair of right eye endpoints, a pair of mouth endpoints, and a nose tip point, and
wherein the predetermined detection area comprises an orbital area of the face and an infraorbital area of the face.

11. The display device of claim 1, wherein the one or more instructions, when executed by the at least one processor, further cause the display device to:
obtain a skin reflection coefficient of the patient based on first image information obtained in an illuminated environment and second image information obtained in an unilluminated environment, the first image information and the second image information being received from the external device, and
transmit illuminance information corresponding to the skin reflection coefficient to the external device.

12. A method for controlling a display device, the method comprising:
identifying an omega shape of a patient and one or more feature points of the patient based on image information received from an external device through a communication circuitry;
based on identifying the omega shape and the one or more feature points, identifying whether the image information includes a detection area associated with biometric information of the patient; and
based on identifying the detection area, adjusting a display parameter of at least one of the image information or a display of the display device.

13. The method of claim 12, further comprising:
based on not identifying the one or more feature points and the omega shape in the image information, transmitting first environment guide information to the external device, wherein the first environment guide information comprises information related to an adjustment of a location of the patient and a posture of the patient, and
based on identifying the omega shape in the image information and not identifying the one or more feature points in the image information:
identifying an environment of a space where the external device is located as a backlit environment, adjusting the display parameter of the display, and performing pre-processing on the image information to make a brightness of an image displayed on the display uniform, and
transmitting second environment guide information to the external device based on reference illuminance information and illuminance information received from the external device through the communication circuitry, wherein the second environment guide information comprises information related to an adjustment to an illuminance of the space.

14. The method of claim 12, further comprising:
identify a center pole of the image information;
identifying, in the image information, a left side of the patient and a right side of the patient based on the center pole;
identifying whether the one or more feature points includes a left feature point on the left side of the patient, and identifying whether the one or more feature points includes a right feature point on the right side of the patient;
based on identifying that only one of the left feature point and the right feature point is included in the one or more feature points:
identifying a feature point, among the left feature point and the right feature point, included in the one or more feature points as a detected feature point, and identifying a feature point, among the left feature point and the right feature point, not included in the one or more feature points as a missing feature point;
generating the missing feature point by mapping the detected feature point to a side from among the left side of the patient and the right side of the patient corresponding to the missing feature point; and
identifying the detection area based on the generated feature point and the detected feature point.

15. The method of claim 12, further comprising:
obtaining a skin reflection coefficient of the patient based on first image information obtained in an illuminated environment and second image information obtained in an unilluminated environment, the first image information and the second image information being received from the external device, and
transmitting illuminance information corresponding to the skin reflection coefficient to the external device.
